# EUROPEAN PATENT SPECIFICATION

(11) **EP 4 226 877 B1**
(45) Date of publication and mention of the grant of the patent: **15.10.2025**
(21) Application number: 22155814.1
(22) Date of filing: 09.02.2022
(51) Int. Cl.: A61B 17/225, A61B 17/22, A61B 17/00, A61B 6/00, A61B 34/00, A61B 90/00, A61B 8/00, A61B 8/08

(54) **SHOCK WAVE DEVICE WITH INTEGRATED ULTRASOUND PROBE**
STOSSWELLENVORRICHTUNG MIT INTEGRIERTER ULTRASCHALLSONDE
DISPOSITIF À ONDES DE CHOC AVEC SONDE À ULTRASONS INTÉGRÉE

(43) Date of publication of application: 16.08.2023
(73) Proprietor: Storz Medical AG, 8274 Tägerwilen (CH)
(72) Inventor: Storz, Rafael, 8280 Kreuzlingen (CH); Marlinghaus, Ernst, 8598 Bottighofen (CH); Görner, Georg, 8597 Landschlacht (CH); Gremlich, Felix, 8280 Kreuzlingen (CH); Kühl, Arvid, 8274 Tägerwilen (CH)
(74) Representative: Lohr, Jöstingmeier & Partner Patent- und Rechtsanwälte mbB

(56) References cited:
- EP-A1- 2 628 456
- EP-A1- 3 875 048
- WO-A2-2008/048708
- US-A1- 2010 080 349
- US-A1- 2016 114 194
- US-A1- 2019 350 685

## Description

### Field of the invention

The invention relates to an extracorporeal shock wave or ultrasound therapy system, e.g., a lithotripsy system or Lithotripter for non-invasive treatment of stones, like e.g., kidney stones, urinary stones, gallstones, or other calculi within a mammal's body using acoustic pulses, or other applications of an extracorporeal shock wave or ultrasound therapy system.

### Description of the related art

Lithotripters which generate high energy pulses to disintegrate concrements in a human body may have shock wave and/or ultrasound sources within a reflector.

For localizing concrements to be treated, X-ray devices or ultrasound localizing systems using an ultrasonic transducer may be used. Such ultrasonic transducers may be handheld by a surgeon, or they may be fixed in a flexible or adjustable holding device. In EP 0 312 847 A1 a lithotripter with an ultrasonic transducer is disclosed, whereby the transducer is mounted at the center of the shock wave source. To allow for scanning a certain region around the focal point of the shock wave source, the transducer is rotatable and tiltable within the shockwave source. The movable transducer requires a significant amount of space within the shockwave source, which degrades the output power and focusing of the shock wave source. Therefore, this is difficult to implement and expensive due to space restrictions within the shockwave source. EP 2 628 456 A1 discloses a shock wave therapy device with dynamic target tracking. US 2010/080349 A1 discloses a breast biopsy device.

### Summary of the invention

The problem to be solved by the invention is to provide an ultrasound and/or shock wave device, which can provide an improved ultrasound locating of concrements, and which has a reduced negative influence on the shock wave source. Solutions of the problem are described in the independent claims. The dependent claims relate to further improvements of the invention.

### Embodiments of the present disclosure

In an embodiment, a shock wave and/or ultrasound device, which may be a lithotripter includes an ultrasound and/or shockwave source which may be within a reflector. Further, an ultrasonic transducer for ultrasonic imaging may be coupled rigidly to the ultrasound and/or shockwave source. It may also be mounted within the reflector. The ultrasonic transducer may be arranged at the center of the ultrasound and/or shockwave source and may further be coaxially to the ultrasound and/or shockwave source. Alternatively, the ultrasonic transducer may be arranged at the center of the reflector and coaxially to the reflector. The ultrasonic transducer may further be oriented towards a focal volume of the shock wave and/or ultrasound device. The ultrasonic transducer may be aligned parallel with the shock wave and/or ultrasound device. The ultrasonic transducer may be configured to generate an image of a part or of a significant portion or all of the focal volume of the shock wave and/or ultrasound device. A focal volume of the shock wave and/or ultrasound device may be indicated in the image of the shock wave and/or ultrasound device. This simplifies targeting or may help to verify automatic targeting. The ultrasonic transducer may be fixedly mounted within the reflector, such that it does not move relative to the reflector.

The ultrasound and/or shockwave source may be suspended on a hexapod drive, such that it can be displaced in at least two or three degrees of freedom and tilted or rotated about one, two or three degrees of freedom. As the hexapod drive allows a free orientation of the source in space, it can basically adjust the ultrasound and/or shockwave source to any required position and orientation.

The ultrasonic transducer moves together with the ultrasound and/or shockwave source. Therefore, the ultrasonic transducer can easily be moved for scanning a larger area by moving the ultrasound and/or shockwave source by means of the hexapod drive.

A controller may be provided to control the hexapod drive for specific movements of the ultrasonic transducer, e.g., for scanning a larger area. A larger area scan may include circular movements of the ultrasonic transducer. Further, the controller may be configured for automatically identifying and/or tracking of a concrement.

In an embodiment, 3D control device e.g., a 3D control device may be provided which allows a manual input for movements in 3D space. Such movements may be translation in two or three axes and tilt about one, two or three axes. In an embodiment, there may be a translation in an X'-Y' plane and rotation about a Z'-Axis, perpendicular to the X'-Y' plane in a coordinate system related to the reflector, where the base of the reflector defines the X'-Y' plane and the center axis of the reflector is along the Z'-Axis. The base of the hexapod may be in the X-Y plane of the system with its perpendicular Z-Axis of the shock wave and/or ultra-sound therapy system. The movements input by the 3D control device may be forwarded to the hexapod drive such that the ultrasonic transducer performs these movements. This allows for an easy movement of the ultrasonic transducer by a surgeon, for example for scanning a patient and/or for locating a concrement. The ultrasonic transducer may be moved by manual inputs to the 3D control device until the concrement is centered. Then the concrement will automatically be within the focal volume of the ultrasound and/or shockwave source. The controller may be configured to limit the movements of the hexapod drive in spatial relations and /or speed such that too wide or too fast movements are prevented. This may help to protect the patient.

The hexapod drive bearing the ultrasound and/or shockwave source is also known as a hexapod platform. Such a hexapod platform also is called a Stewart platform. Basically, it is a type of parallel manipulator or parallel robot that has six linear actuators, which may be hydraulic or pneumatic jacks or electric linear actuators. Examples of electric linear actors are motors coupled to a belt or a spindle to perform a linear movement. These linear actuators are connected in pairs to three mounting positions at a base, crossing over to three mounting positions at the ultrasound and/or shockwave source. Each connection of a linear actuator to either the base or the ultrasound and/or shockwave source may include a universal joint, also called a cardan joint or a ball joint. By variation of the length of the linear actuators, the ultrasound and/or shockwave source can be moved in six degrees of freedom with respect to the base. There are three degrees of translation and three degrees of rotation. Although the use of six linear actuators is preferred, a lower number of actuators may be used, e.g., like in a delta robot with three actuators.

The hexapod drive allows to adjust the position of an ultrasound and/or shockwave source together with the ultrasonic transducer relative to a patient's body and/or the X-ray system. Positioning of the ultrasound and/or shockwave source may be done automatically or by manual control. An automatic control may allow quick adjustment and it may also allow to store and retrieve preconfigured settings.

Such a hexapod drive is a very robust and mechanical stiff support or suspension of the ultrasound and/or shockwave source. Therefore, it can withstand high forces from the patient body, the weight of the ultrasound and/or shockwave source and dynamic loads which occur when shockwave pulses are generated. Further, a hexapod drive can be moved quickly. Therefore, a stable positioning of the ultrasound and/or shockwave source is achieved providing the additional ability to quickly correct deviations and movements by the patient.

A hexapod drive may allow movement in all 6 degrees of freedom. This allows for a precise adjustment of the position of the focal volume of the ultrasound and/or shockwave source and/or the path of the ultrasound and/or shockwave though the body of the patient.

There may be constraints in the movements, e.g., for safety reasons. In an example, the movement in Z direction may be limited to avoid injuring of the patient.

The ultrasound and/or shockwave source is oriented in a cartesian coordinate system as follows: a y-axis may be a longitudinal axis through the center of a patient table. An x-axis may be orthogonal to the y-axis and in the plane of the table surface. A z-axis is orthogonal to the plane of the table surface and therefore orthogonal to the x-axis and the y-axis and in a direction upward from the table. There may also be a first rotation around the x-axis, a second rotation and a third rotation around the z-axis. A positive rotation may be a clockwise rotation in a direction of a positive axis.

The shock wave or ultrasound therapy system may include a system controller which may control at least the hexapod drive of the ultrasound and/or shockwave source.

The ultrasound and/or shockwave source may be of any type suitable for generating shock waves. It may include a shock wave generator and/or transducer, which may include at least one of a coil, a spark gap, or a Piezo transducer. The shock wave generator/transducer may be partially enclosed by a reflector. Depending on the type of transducer, the reflector may have a parabolic or half-elliptic shape. The ultrasound and/or shockwave source may have a focal volume which is distant from the ultrasound and/or shockwave source and normally around a center axis of the ultrasound and/or shockwave source. The focal volume may be defined as a volume, where the maximum shock wave intensity is maintained with a deviation of maximal -3 dB or -6 dB. If the focal volume is defined with a 6 dB deviation, the pressure at the limit of the zone is half of the maximum pressure inside the zone. The focal volume may have an elliptical shape with a length in an axial direction (defined by the center axis) of the ultra-sound and/or shockwave source axis of 10 to 15 cm and a diameter between 5 and 15 mm. The focal volume normally is spaced from the shock wave generator and/or transducer.

The patient table may have a basically planar surface defining a longitudinal axis. It is configured for accommodating a patient. The ultrasound and/or shockwave source may be mounted below the patient table. In general, a shockwave source may be mounted in alternative ways, e.g., on a stand or support.

The base may be standing on a floor directly or by a stand or it may be attached to a floor. The base may also hold the table.

The surgical and therapeutic methods disclosed are not claimed.

A method of operating an ultrasound and/or shockwave source being suspended on a hexapod drive includes the steps of:
receiving input of an operator by a 3D control device,
forwarding the input signals to a system controller, and
controlling the hexapod drive to perform movements as directed by the 3D control device.

In an embodiment, the method of operating an ultrasound and/or shockwave source includes:
receiving a five or six degrees of freedom input from the 3D control device and
performing five degrees of freedom movements by the hexapod drive.

The five or six degrees of freedom may include translation in all three axes and tilt/rotation about the axes in the X-Y plane.

### Description of Drawings

In the following the invention will be described by way of example, without limitation of the general inventive concept, on examples of embodiment with reference to the drawings. The scope of the invention is defined by the wording of the claims.
Figure 1 shows an embodiment of a lithotripsy system.
Figure 2 shows an embodiment in a schematic view.
Figure 3 shows a side view.
Figure 4 shows a more detailed view of the control panel.

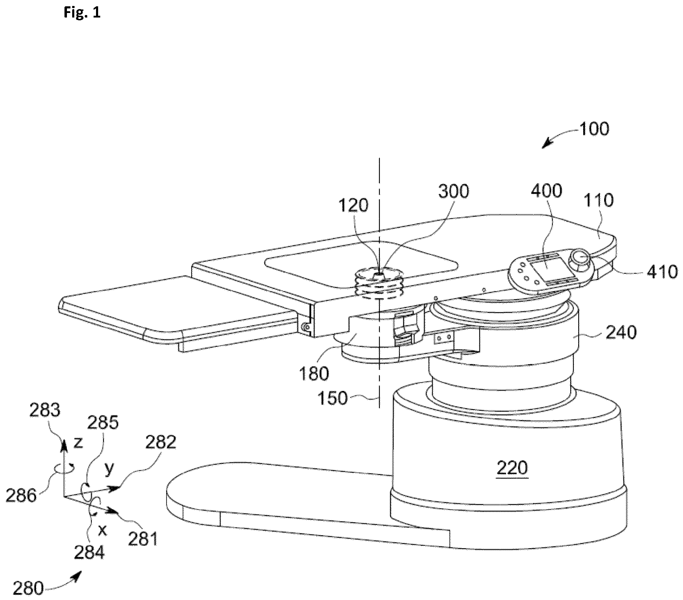

In Figure 1, a first embodiment of a lithotripsy system is shown. The invention works with any ultrasound and/or shockwave treatment system.

An extra-corporal ultrasound and/or shockwave lithotripsy system for non-invasive treatment of stones 100 comprises a patient table 110, an ultra-sound and/or shockwave source 120 together with an ultrasonic transducer 300 for ultrasonic imaging. The ultrasound and/or shockwave source 120 is mounted to a hexapod drive 180. The hexapod drive 180 may further be held by a stand 220. The hexapod drive 180 allows fine positioning of the ultrasound and/or shockwave source 120 in multiple axes relative to the patient table 110 and therefore relative to the patient (not shown in this figure). The ultrasound and/or shockwave source 120 has a focal volume which moves together with the source. In an embodiment the distance of the focal volume to the source may be modified. The patient table 110 is based on a stand 220 which may stand on a floor. This stand may be the same or a different than the stand holding the shockwave source. The table 110 may be held by a positioning device 240 to move the table relative to the ultrasound and/or shockwave source 120 together with the hexapod drive 180. A movement of the patient table may be coarse positioning which may be limited to a displacement in 3 axes.

The table 110 may have a flat surface for accommodating a patient who is not shown here.

To describe the relative movement of the table 110, and the ultrasound and/or shockwave source 120, a cartesian coordinate system 280 may be used. There is a y-axis 282, which may be a longitudinal axis 112 through the center of the table. Furthermore, there is an x-axis 281 orthogonal to the y-axis and in the plane of the table surface. A z-axis 283 is orthogonal to the plane of the table surface and therefore orthogonal to the x-axis and the y-axis. There may also be a first rotation 284 around the x-axis, a second rotation 285 and a third rotation 286 around the z-axis. A positive rotation may be a clockwise rotation in a view along a positive axis.

A hexapod drive 180 may allow movement in all these 6 degrees of freedom. This allows for a precise adjustment of the position of the focal volume of the ultra-sound and/or shockwave source 120. The patient table may be positioned for a slow and coarse adjustment in 3 degrees of translation for larger distances, but normally no rotation, whereas the hexapod drive provides a comparatively quick adjustment in 3 degrees of translation and 3 degrees of rotation. The movement distances of the table may be larger than the movement distances of the hexapod drive. An ultrasound device may at least be tiltable to perform a second rotation 285 around an axis parallel to the y-axis 282. It may also be tiltable around an axis parallel to the Y axis. It may further be translated in a plane defined by the X and Y axis. This means, it may be moved on the floor which is also in the X-Y plane. Further, it may be rotated along the Z-axis of the shockwave source 120.

For control of the movement of the ultrasound and/or shockwave source 120 relative to the patient table 110, a control panel 400 may be provided, which may include a 3D control device 410, e.g., a control button.

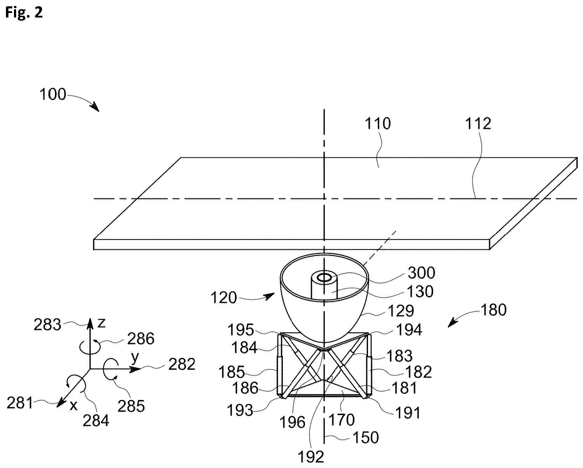

Figure 2 shows an embodiment in a schematic view. An embodiment with an ultrasound and/or shockwave source 120 may include an ultrasonic transducer for ultrasonic imaging 300 mounted to a hexapod drive 180. Below the table 110, the ultrasound and/or shockwave source 120 is shown. It may have a shock wave generator 130 which may be a include a cylindrical as shown herein and which is at least partially enclosed by a reflector 129. Further, the center axis 150 is marked as a dashed line.

A shock wave generator 130 may be held within a reflector 129. In this embodiment, the shock wave generator 130 may include a coil. Centered to the shock wave generator 130 an ultrasonic transducer for ultrasonic imaging 300 may be provided. It may be aligned with the center axis of the ultrasound and/or shockwave source. Such an ultrasonic transducer is basically mounted together with the ultrasound and/or shockwave source 120 at the hexapod drive 180, such that both components are moved together. The ultrasonic transducer may also be mounted laterally, with an offset to the center axis or under an angle to the center axis.

The ultrasound and/or shockwave source 120 which may have a center axis 150 is supported by and/or suspended on a hexapod drive 180. Basically, it is a type of parallel manipulator or parallel robot that has six linear actuators 181 - 186, which may be hydraulic or pneumatic jacks or electric linear actuators. These linear actuators are connected in pairs to three mounting positions 191, 192, 193 at a base 170, crossing over to three mounting positions 194, 195, 196 at the ultra-sound and/or shockwave source 120. Each connection of a linear actuator to either the base or the ultrasound and/or shockwave source may include a universal joint, also called a cardan joint or a ball joint. By variation of the length of the linear actuators, the ultrasound and/or shockwave source can be moved in six degrees of freedom with respect to the base. There are three degrees of translation, parallel to at least one X-axis 281, Y-axis 282, Z-axis 283 and three degrees of tilt or rotation including a first tilt 284 around an axis parallel to X-axis 281, a second tilt 285 around an axis parallel to Y-axis 282, a third tilt 285 around an axis parallel to Z-axis 283.

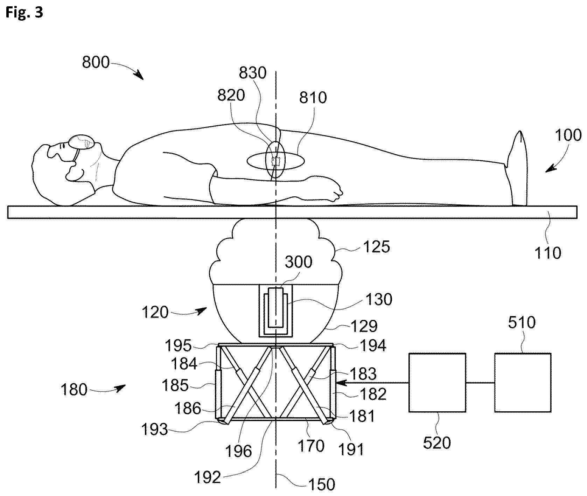

Figure 3 shows a side view. A shock wave or ultrasound device, which may be a lithotripter 100 may include a patient table 110 and an ultrasound and/or shockwave source 120. The ultrasound and/or shockwave source 120 which is shown in a sectional view may be arranged below the patient table 110, such that a patient 800 may be accommodated on top of the patient table. The patient may have a kidney 810 with a kidney stone 820.

A shock wave generator 130 may be held within a reflector 129. In this embodiment, the shock wave generator 130 may include a coil. Centered to the shock wave generator 130 an ultrasonic transducer for ultrasonic imaging 300 may be provided. It may be aligned with the center axis of the ultrasound and/or shockwave source. Such an ultrasonic transducer for ultrasonic imaging may basically be mounted together with the ultrasound and/or shockwave source 120 at the hexapod drive 180, such that both components may be moved together. The ultrasonic transducer for ultrasonic imaging may be configured to generate an image of a part or all of a focal volume 830 of the shock wave and/or ultrasound source 120.

A system controller 510 may be provided to control all system functions. It may also receive input from the control panel 400 and provide user feedback via the control panel. A hexapod control 520 may receive input from the system controller 510 and control the individual actuators of the hexapod drive.

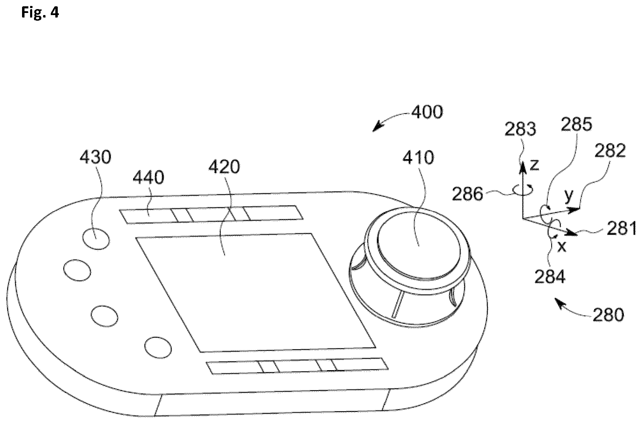

Figure 4 shows a more detailed view of the control panel 400. Such a control panel may include a 3D control device 410, which may be configured to forward manual input to the system controller 510, which then controls the hexapod drive 180 to move as directed by the 3D control device 410. The control panel may also include at least one of a display 420, buttons 430 and indicators 440. A joystick - arrangement instead of a 3D control button as a 3D control device may also serve as input device in 6 or less degrees of freedom. Alternatively, 3D control signals may be derived by a 3D camera detecting a marker in space. Such a marker may be attached to a handle or other control element, which may be moved in 3D space by an operator.

### List of reference numerals

- 100: shock wave and/or ultrasound therapy system
- 110: patient table
- 112: longitudinal axis
- 120: ultrasound and/or shockwave source
- 129: reflector
- 130: shock wave generator
- 150: center axis
- 170: base
- 180: hexapod drive
- 181-186: linear actuators
- 191-193: mounting positions at base
- 194-196: mounting positions at ultrasound and/or shockwave source
- 220: stand
- 240: positioning device
- 280: cartesian coordinate system
- 281: x-axis
- 282: y-axis
- 283: z-axis
- 284: tilt around the x-axis
- 285: tilt around the y-axis
- 286: tilt around the z-axis
- 300: ultrasonic transducer for ultrasonic imaging
- 400: control panel
- 410: 3D control device
- 420: display
- 430: buttons
- 440: indicators
- 510: system controller

- 520: hexapod control
- 800: patient
- 810: kidney
- 820: kidney stone
- 830: focal volume

## Claims

1. A shock wave and/or ultrasound therapy system (100) comprising an ultra-sound and/or shockwave source (120) further comprising an ultrasonic transducer for ultrasonic imaging (300)
**characterized in, that**
the ultrasound and/or shockwave source (120) is suspended on a hexapod drive (180), and
the ultrasonic transducer for ultrasonic imaging (300) is coupled rigidly to the ultrasound and/or shockwave source (120) and/or the hexapod drive (180) such that movements of the hexapod drive (180) are directly coupled to the ultrasonic transducer.

2. The shock wave and/or ultrasound therapy system (100) according to claim 1,
**characterized in, that**
the shock wave and/or ultrasound therapy system (100) further comprises a reflector (129) coupled to the hexapod drive (180), wherein the ultrasonic transducer for ultrasonic imaging is coupled to the reflector (129).

3. The shock wave and/or ultrasound therapy system (100) according to any of the preceding claims,
**characterized in, that**
the ultrasonic transducer for ultrasonic imaging (300) is arranged:
at the center of the ultrasound and/or shockwave source (120) and coaxially to the ultrasound and/or shockwave source (120) or
at the center of the reflector (129) and coaxially to the reflector (129).

4. The shock wave and/or ultrasound therapy system (100) according to any of claims 1 or 2,
**characterized in, that**
the ultrasonic transducer for ultrasonic imaging (300) is aligned parallel with the ultrasound and/or shockwave source (120).

5. The shock wave and/or ultrasound therapy system (100) according to any of the preceding claims,
**characterized in, that**
the ultrasonic transducer for ultrasonic imaging (300) is oriented towards a focal volume of the shock wave and/or ultrasound device.

6. The shock wave and/or ultrasound therapy system (100) according to any of the preceding claims,
**characterized in, that**
the ultrasonic transducer for ultrasonic imaging (300) is configured to generate an image of a part or all of a focal volume (830) of the shock wave and/or ultrasound source (120) wherein a focal volume of the shock wave and/or ultrasound device may be indicated in the image.

7. The shock wave and/or ultrasound therapy system (100) according to any of the preceding claims,
**characterized in, that**
a 3D control device is provided, which allows a manual input for movements in 3D space of the ultrasound and/or shockwave source (120) together with the ultrasonic transducer for ultrasonic imaging (300).

8. The shock wave and/or ultrasound therapy system (100) according to any of the preceding claims,
**characterized in, that**
the hexapod drive (180) includes six linear actuators (181-186) which are attached in pairs to three positions (191-193) at the base (170), crossing over to three mounting positions (194-196) at the ultrasound and/or shockwave source (120).

9. The shock wave and/or ultrasound therapy system (100) according to the preceding claim,
**characterized in, that**
each connection of the linear actuators (181-186) to either the base (170) or the ultrasound and/or shockwave source (120) includes a universal joint or a ball joint.

10. The shock wave and/or ultrasound therapy system (100) according to any of the preceding claims,
**characterized in, that**
the linear actuators (181-186) include at least one of hydraulic or pneumatic jacks or electric linear actuators.

11. The shock wave and/or ultrasound therapy system (100) according to any of the preceding claims,
**characterized in, that**
the hexapod drive (180) is configured to provide movement with at least five degrees of freedom including displacement along three orthogonal axes (281, 282, 283) and at least two degrees of tilt.

12. The shock wave and/or ultrasound therapy system (100) according to any of the preceding claims,
**characterized in, that**
the system further includes a patient table (110), and
the shock wave and/or ultrasound therapy system (100) is arranged in a cartesian coordinate system with a y-axis (282) being a longitudinal axis through the center of the patient table (110), an x-axis (281) being orthogonal to the y-axis and in the plane of a surface of the patient table (110), a z-axis (283) being orthogonal to the plane of the surface of the patient table (110), and therefore orthogonal to the x-axis and the y-axis and in a direction upward from the table.

13. The shock wave and/or ultrasound therapy system (100) according to claim 12,
**characterized in, that**
the ultrasound and/or shockwave source (120) is configured to generate shock waves propagating in a direction to the patient table (110) above the ultrasound and/or shockwave source (120) and having a focal volume (350) above the patient table (110).

14. A method of operating an ultrasound system or an ultrasound and shockwave system in order to scan a patient and/or to localize a concrement, wherein said ultrasound and/or shockwave source being suspended on a hexapod drive (180), the method includes the steps of:
receiving input of an operator by a 3D control device (410);
forwarding the input signals to a system controller, and
controlling the hexapod drive (180) to perform movements as directed by the 3D control device (410).

15. The method of claim 14 including: receiving at least five degrees of freedom input from the 3D control device (410) and performing at least five degrees of freedom movements by the hexapod drive (180).

## Patentansprüche

1. Ein Stoßwellen- und/oder Ultraschalltherapiesystem (100), das eine Ultraschall- und/oder Stoßwellenquelle (120) umfasst, die weiterhin einen Ultraschallwandler zur Ultraschallbildgebung (300) umfasst,
**dadurch gekennzeichnet, dass**
die Ultraschall- und/oder Stoßwellenquelle (120) an einem Hexapodantrieb (180) aufgehängt ist, und
der Ultraschallwandler zur Ultraschallbildgebung (300) starr mit der Ultraschall- und/oder Stoßwellenquelle (120) und/oder dem Hexapodantrieb (180) gekoppelt ist, so dass Bewegungen des Hexapodantriebs (180) direkt mit dem Ultraschallwandler gekoppelt sind.

2. Das Stoßwellen- und/oder Ultraschalltherapiesystem (100) nach Anspruch 1,
**dadurch gekennzeichnet, dass**
das Stoßwellen- und/oder Ultraschalltherapiesystem (100) weiterhin einen mit dem Hexapodantrieb (180) gekoppelten Reflektor (129) umfasst, wobei der Ultraschallwandler zur Ultraschallbildgebung mit dem Reflektor (129) gekoppelt ist.

3. Das Stoßwellen- und/oder Ultraschalltherapiesystem (100) nach einem der vorhergehenden Ansprüche,
**dadurch gekennzeichnet, dass**
der Ultraschallwandler zur Ultraschallbildgebung (300) angeordnet ist:
im Zentrum der Ultraschall- und/oder Stoßwellenquelle (120) und koaxial zur Ultraschall- und/oder Stoßwellenquelle (120) oder
im Zentrum des Reflektors (129) und koaxial zum Reflektor (129).

4. Das Stoßwellen- und/oder Ultraschalltherapiesystem (100) nach einem der Ansprüche 1 oder 2,
**dadurch gekennzeichnet, dass**
der Ultraschallwandler zur Ultraschallbildgebung (300) parallel zur Ultraschall- und/oder Stoßwellenquelle (120) ausgerichtet ist.

5. Das Stoßwellen- und/oder Ultraschalltherapiesystem (100) nach einem der vorhergehenden Ansprüche,
**dadurch gekennzeichnet, dass**
der Ultraschallwandler zur Ultraschallbildgebung (300) auf ein Fokusvolumen der Stoßwellen- und/oder Ultraschallvorrichtung ausgerichtet ist.

6. Das Stoßwellen- und/oder Ultraschalltherapiesystem (100) nach einem der vorhergehenden Ansprüche,
**dadurch gekennzeichnet, dass**
der Ultraschallwandler für die Ultraschallbildgebung (300) dazu konfiguriert ist, ein Bild eines Teils oder des gesamten Fokusvolumens (830) der Stoßwellen- und/oder Ultraschallquelle (120) zu erzeugen, wobei ein Fokusvolumen der Stoßwellen- und/oder Ultraschallvorrichtung im Bild angezeigt werden kann.

7. Das Stoßwellen- und/oder Ultraschalltherapiesystem (100) nach einem der vorhergehenden Ansprüche,
**dadurch gekennzeichnet, dass**
eine 3D-Steuervorrichtung vorgesehen ist, die eine manuelle Eingabe für Bewegungen im 3D-Raum der Ultraschall- und/oder Stoßwellenquelle (120) zusammen mit dem Ultraschallwandler zur Ultraschallbildgebung (300) ermöglicht.

8. Stoßwellen- und/oder Ultraschalltherapiesystem (100) nach einem der vorhergehenden Ansprüche,
**dadurch gekennzeichnet, dass**
der Hexapodantrieb (180) sechs Linearantriebe (181-186) umfasst, die paarweise an drei Positionen (191-193) an der Basis (170) befestigt sind, über drei Befestigungspositionen (194-196) an der Ultraschall- und/oder Stoßwellenquelle (120) verlaufend.

9. Das Stoßwellen- und/oder Ultraschalltherapiesystem (100) nach dem vorhergehenden Anspruch,
**dadurch gekennzeichnet, dass**
jede Verbindung der Linearaktuatoren (181-186) entweder mit der Basis (170) oder der Ultraschall- und/oder Stoßwellenquelle (120) ein Universalgelenk oder ein Kugelgelenk umfasst.

10. Das Stoßwellen- und/oder Ultraschalltherapiesystem (100) nach einem der vorhergehenden Ansprüche,
**dadurch gekennzeichnet, dass**
die Linearaktuatoren (181-186) mindestens eines von hydraulischen oder pneumatischen Zylindern oder elektrischen Linearaktuatoren umfassen.

11. Das Stoßwellen- und/oder Ultraschalltherapiesystem (100) nach einem der vorhergehenden Ansprüche,
**dadurch gekennzeichnet, dass**
der Hexapodantrieb (180) dazu konfiguriert ist, Bewegung mit mindestens fünf Freiheitsgraden bereitzustellen, einschließlich Verschiebung entlang dreier orthogonaler Achsen (281, 282, 283) und Neigung von mindestens zwei Grad.

12. Das Stoßwellen- und/oder Ultraschalltherapiesystem (100) nach einem der vorhergehenden Ansprüche,
**dadurch gekennzeichnet, dass**
das System weiterhin einen Patiententisch (110) umfasst, und
das Stoßwellen- und/oder Ultraschalltherapiesystem (100) in einem kartesischen Koordinatensystem angeordnet ist, wobei eine y-Achse (282) eine Längsachse durch die Mitte eines Patiententisches (110) ist, eine x-Achse (281) orthogonal zur y-Achse und in der Ebene einer Oberfläche des Patiententisches (110) ist, eine z-Achse (283) orthogonal zur Ebene der Oberfläche des Patiententisches (110) und daher orthogonal zur x-Achse und zur y-Achse und in einer Richtung nach oben vom Tisch ist.

13. Das Stoßwellen- und/oder Ultraschalltherapiesystem (100) nach einem der vorhergehenden Ansprüche,
**dadurch gekennzeichnet, dass**
die Ultraschall- und/oder Stoßwellenquelle (120) dazu konfiguriert ist, Stoßwellen zu erzeugen, die sich in Richtung eines Patiententisches (110) über der Ultraschall- und/oder Stoßwellenquelle (120) ausbreiten und ein Fokusvolumen (350) über dem Patiententisch (110) aufweisen.

14. Ein Verfahren zum Betreiben eines Ultraschallsystems oder eines Ultraschall- und Stoßwellensystems, um
einen Patienten zu scannen und/oder ein Konkrement zu lokalisieren, wobei die Ultraschall- und/oder Stoßwellenquelle an einem Hexapodantrieb (180) aufgehängt ist, wobei das Verfahren die folgenden Schritte umfasst:
Empfangen von Eingaben eines Bedieners durch eine 3D-Steuervorrichtung (410);
Weiterleiten der Eingangssignale an eine Systemsteuerung, und
Steuern des Hexapodantriebs (180), um Bewegungen gemäß den Anweisungen der 3D-Steuervorrichtung (410) auszuführen.

15. Das Verfahren nach Anspruch 14, umfassend: Empfangen von Eingaben von mindestens fünf Freiheitsgraden von der 3D-Steuervorrichtung (410) und Ausführen von Bewegungen in mindestens fünf Freiheitsgraden durch den Hexapodantrieb (180).

## Revendications

1. Système de thérapie par ondes de choc et/ou ultrasons (100) comprenant une source d'ultrasons et/ou d'ondes de choc (120) comprenant en outre un transducteur ultrasonore pour imagerie ultrasonore (300)
**caractérisé en ce que**
la source d'ultrasons et/ou d'ondes de choc (120) est suspendue sur un entraînement hexapode (180), et
le transducteur ultrasonore pour imagerie ultrasonore (300) est couplé de manière rigide à la source d'ultrasons et/ou d'ondes de choc (120) et/ou à l'entraînement hexapode (180) de telle sorte que les mouvements de l'entraînement hexapode (180) sont directement couplés au transducteur ultrasonore.

2. Système de thérapie par ondes de choc et/ou ultrasons (100) selon la revendication 1,
**caractérisé en ce que**
le système de thérapie par ondes de choc et/ou ultrasons (100) comprend en outre
un réflecteur (129) couplé à l'entraînement hexapode (180), dans lequel le transducteur ultrasonore pour imagerie ultrasonore est couplé au réflecteur (129).

3. Système de thérapie par ondes de choc et/ou ultrasons (100) selon l'une quelconque des revendications précédentes,
**caractérisé en ce que**
le transducteur ultrasonore pour imagerie ultrasonore (300) est agencé :
au centre de la source d'ultrasons et/ou d'ondes de choc (120) et coaxialement à la source d'ultrasons et/ou d'ondes de choc (120) ou
au centre du réflecteur (129) et coaxialement au réflecteur (129).

4. Système de thérapie par ondes de choc et/ou ultrasons (100) selon l'une quelconque des revendications 1 ou 2,
**caractérisé en ce que**
le transducteur ultrasonore pour imagerie ultrasonore (300) est aligné parallèlement à la source d'ultrasons et/ou d'ondes de choc (120).

5. Système de thérapie par ondes de choc et/ou ultrasons (100) selon l'une quelconque des revendications précédentes,
**caractérisé en ce que**
le transducteur ultrasonore pour imagerie ultrasonore (300) est orienté vers un volume focal du dispositif à ondes de choc et/ou à ultrasons.

6. Système de thérapie par ondes de choc et/ou ultrasons (100) selon l'une quelconque des revendications précédentes,
**caractérisé en ce que**
le transducteur ultrasonore pour imagerie ultrasonore (300) est configuré pour générer une image d'une partie ou de la totalité d'un volume focal (830) de la source d'ondes de choc et/ou d'ultrasons (120) dans lequel un volume focal du dispositif à ondes de choc et/ou à ultrasons peut être indiqué dans l'image.

7. Système de thérapie par ondes de choc et/ou ultrasons (100) selon l'une quelconque des revendications précédentes,
**caractérisé en ce que**
un dispositif de commande 3D est prévu, lequel permet une entrée manuelle pour des mouvements dans un espace 3D de la source d'ultrasons et/ou d'ondes de choc (120) conjointement avec le transducteur ultrasonore pour imagerie ultrasonore (300).

8. Système de thérapie par ondes de choc et/ou ultrasons (100) selon l'une quelconque des revendications précédentes,
**caractérisé en ce que**
l'entraînement hexapode (180) inclut six actionneurs linéaires (181-186) qui sont fixés par paires à trois positions (191-193) au niveau de la base (170), se croisant en trois positions de montage (194-196) au niveau de la source d'ultrasons et/ou d'ondes de choc (120).

9. Système de thérapie par ondes de choc et/ou ultrasons (100) selon la revendication précédente,
**caractérisé en ce que**
chaque connexion des actionneurs linéaires (181-186) soit à la base (170) soit à la source d'ultrasons et/ou d'ondes de choc (120) inclut un joint universel ou un joint à rotule.

10. Système de thérapie par ondes de choc et/ou ultrasons (100) selon l'une quelconque des revendications précédentes,
**caractérisé en ce que**
les actionneurs linéaires (181-186) incluent au moins un parmi des vérins hydrauliques ou pneumatiques ou des actionneurs linéaires électriques.

11. Système de thérapie par ondes de choc et/ou ultrasons (100) selon l'une quelconque des revendications précédentes,
**caractérisé en ce que**
l'entraînement hexapode (180) est configuré pour fournir un mouvement avec au moins cinq degrés de liberté incluant un déplacement le long de trois axes orthogonaux (281, 282, 283) et au moins deux degrés d'inclinaison.

12. Système de thérapie par ondes de choc et/ou ultrasons (100) selon l'une quelconque des revendications précédentes,
**caractérisé en ce que**
le système inclut en outre une table de patient (110), et
le système de thérapie par ondes de choc et/ou ultrasons (100) est agencé dans un système de coordonnées cartésiennes, un axe y (282) étant un axe longitudinal passant par le centre de la table de patient (110), un axe x (281) étant orthogonal à l'axe y et dans le plan d'une surface de la table de patient (110), un axe z (283) étant orthogonal au plan de la surface de la table de patient (110), et donc orthogonal à l'axe x et à l'axe y et dans une direction vers le haut à partir de la table.

13. Système de thérapie par ondes de choc et/ou ultrasons (100) selon la revendication 12,
**caractérisé en ce que**
la source d'ultrasons et/ou d'ondes de choc (120) est configurée pour générer des ondes de choc se propageant dans une direction vers la table de patient (110) au-dessus de la source d'ultrasons et/ou d'ondes de choc (120) et ayant un volume focal (350) au-dessus de la table de patient (110).

14. Procédé de fonctionnement d'un système à ultrasons ou d'un système à ultrasons et à ondes de choc afin de balayer un patient et/ou de localiser une concrétion, dans lequel ladite source d'ultrasons et/ou d'ondes de choc est suspendue sur un entraînement hexapode (180), le procédé inclut les étapes de :
réception d'une entrée d'un opérateur par un dispositif de commande 3D (410) ;
transfert des signaux d'entrée à un dispositif de commande de système, et
commande de l'entraînement hexapode (180) pour réaliser des mouvements comme indiqué par le dispositif de commande 3D (410).

15. Procédé selon la revendication 14, incluant : la réception d'au moins une entrée à cinq degrés de liberté provenant du dispositif de commande 3D (410) et la réalisation d'au moins des mouvements à cinq degrés de liberté par l'entraînement hexapode (180).
